**Europäisches Patentamt**

**European Patent Office**

**Office européen des brevets**

(19)

(11) **EP 1 212 961 A1**

(12) # DEMANDE DE BREVET EUROPEEN

(43) Date de publication:
**12.06.2002 Bulletin 2002/24**

(51) Int Cl.⁷: **A45D 44/00**

(21) Numéro de dépôt: **01403167.8**

(22) Date de dépôt: **07.12.2001**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **08.12.2000 FR 0016000**

(71) Demandeur: **L'OREAL**
**75008 Paris (FR)**

(72) Inventeurs:
• **De Rigal, Jean**
  **Gressy, 77410 Claye Souilly (FR)**
• **Dauga, Christophe**
  **92300 Levallois-Perret (FR)**

(74) Mandataire: **Tanty, François**
**Nony & Associés,**
**3, rue de Penthièvre**
**75008 Paris (FR)**

(54) **Nuancier**

(57) Nuancier (1) comportant au moins un modèle de comparaison (4) reproduisant la couleur d'un élément kératinique, notamment de la peau, le ou chaque modèle de comparaison étant réalisé au moyen de pigments et/ou de colorants choisis de telle sorte que son spectre de réflectance présente un profil suffisamment proche de celui de l'élément kératinique dont il reproduit la couleur pour que ce dernier et le modèle de comparaison correspondant apparaissent, pour un observateur, de la même couleur sous au moins deux illuminants différents.

FIG.1

## Description

La présente invention concerne un nuancier regroupant une pluralité de modèles de comparaison destinés chacun à reproduire fidèlement la couleur d'un élément kératinique tel que la peau, les lèvres, les ongles ou les cheveux.

On sait que la couleur de la peau est due à la rétrodiffusion de la lumière par l'épiderme et par le derme où se trouvent respectivement le pigment mélanine en quantité variable et l'hémoglobine.

L'oeil humain est extrêmement sensible à l'apparence de la peau, notamment à sa couleur, laquelle est souvent le reflet des états physique et psychique de l'individu.

Il existe un besoin pour disposer d'un nuancier capable de reproduire fidèlement la couleur de la peau, des lèvres, des ongles ou des cheveux, dans des environnements différents, en particulier sous des éclairages différents.

La présente invention vise notamment à répondre à ce besoin.

Elle y parvient grâce à un nuancier comportant au moins un modèle de comparaison ayant un spectre de réflectance et étant configuré de manière à simuler sensiblement la couleur d'un élément kératinique ayant un spectre de réflectance, nuancier dans lequel ledit au moins un modèle de comparaison comporte au moins l'un d'un pigment et d'un colorant sélectionné de telle manière que le spectre de réflectance du modèle de comparaison soit sensiblement similaire au spectre de réflectance de l'élément kératinique, de sorte que ledit au moins un modèle de comparaison et l'élément kératinique apparaissent pour un observateur comme ayant sensiblement la même couleur pour au moins deux illuminants différents. Ledit au moins un modèle de comparaison peut être configuré de manière à être présent sur l'emballage d'un produit.

Le nuancier peut comporter au moins un modèle de comparaison configuré pour simuler sensiblement la couleur de la peau.

L'invention a ainsi pour objet un nuancier comportant au moins un modèle de comparaison reproduisant la couleur d'un élément kératinique, notamment de la peau, ce nuancier étant caractérisé par le fait que le ou chaque modèle de comparaison est réalisé au moyen de pigments et/ou de colorants choisis de telle sorte que son spectre de réflectance présente un profil suffisamment proche de celui de l'élément kératinique dont il reproduit la couleur pour que ce dernier et le modèle de comparaison correspondant apparaissent, pour un observateur, de la même couleur sous au moins deux illuminants différents.

Un utilisateur du nuancier tel qu'une esthéticienne, une clinicienne ou un dermatologue, ou une personne sans qualification particulière en cosmétique, peut sélectionner sous un illuminant donné un modèle de comparaison, correspondant par exemple à une couleur de peau que l'on cherche à évaluer, tout en étant assuré que le résultat de la sélection reste valable sous un autre illuminant.

De préférence, chaque modèle de comparaison reproduit fidèlement la couleur de l'élément kératinique au moins sous deux des illuminants D65 (lumière du jour), D50 et A (lampe à incandescence).

Selon un aspect de l'invention, l'écart de couleur induit par le changement d'illuminant est inférieur à 4 et de préférence encore inférieur à 2.

L'évaluation de la couleur de la peau ou de la couleur d'un élément kératinique autre que la peau est ainsi facilitée, puisqu'il n'est pas nécessaire d'utiliser au moment de la comparaison un système d'éclairage ayant un spectre d'émission spécifique.

Dans une mise en oeuvre préférée de l'invention, le spectre de réflectance de chaque modèle de comparaison reste proche de celui de l'élément kératinique correspondant dans un domaine spectral s'étendant de préférence de 400 à 800 nm.

De préférence,

$$1/N(\lambda) \sum_{\lambda} \left| I_R^{MOD}(\lambda) - I_R^{REF}(\lambda) \right| / I_R^{REF}(\lambda),$$

où $I_R^{MOD}(\lambda)$ est l'intensité lumineuse réfléchie à la longueur d'onde $\lambda$ pour le modèle de comparaison et $I_R^{REF}(\lambda)$ est l'intensité lumineuse réfléchie à la longueur d'onde $\lambda$ pour l'élément kératinique,

est inférieur ou égal à 0,1 de préférence inférieur à 0,05 et de préférence encore < 0,01.

Dans le cas notamment où le nuancier comporte des modèles de comparaison reproduisant des couleurs de peau, les modèles de comparaison présentent avantageusement des teintes différentes, les angles de teinte étant de préférence compris entre 40° et 70°, et de préférence entre 46° et 64° dans l'espace colorimétrique CIEL*C*h 1976.

Le nuancier comporte par exemple au moins dix catégories de modèles de comparaison présentant chacune une teinte déterminée, différente de celle des autres catégories.

Les modèles de comparaison peuvent aussi présenter des clartés différentes, les niveaux de clarté (L* dans l'espace colorimétrique CIEL*C*h 1976) étant de préférence compris entre 34 et 75.

Le nuancier comporte par exemple au moins cinq catégories de modèles de comparaison présentant chacune une teinte déterminée, différente de celle des autres catégories.

Dans une mise en oeuvre préférée de l'invention, le nuancier comporte cinquante couleurs correspondant à la combinaison de cinq teintes et de dix niveaux de clarté.

De préférence, l'écart de couleur global $\Delta E^*C^*h$ .94 mesuré dans l'espace colorimétrique CIEL*C*h 1976, entre deux modèles de comparaison correspondant à des couleurs de peau voisines, est constant, cet écart étant de préférence compris entre 1 et 40, et de préférence entre 1 et 20 et de préférence encore voisin de 4.

Au sein d'un même modèle de comparaison, la couleur peut être uniforme et constante sur l'ensemble de la surface du modèle de comparaison ou être non uniforme, afin d'imiter la texture de la peau par exemple.

Un modèle de comparaison peut ainsi recevoir deux revêtements colorés de couleurs différentes, l'ensemble produisant une couleur moyenne pour l'oeil.

Le choix des pigments et/ou colorants utilisés pourra être effectué le cas échéant en fonction de l'incidence sur la couleur finale des juxtapositions ou superpositions des différentes couleurs sur le modèle de comparaison.

Un modèle de comparaison peut aussi recevoir un revêtement coloré sur un support non lisse dont le relief est choisi de manière à imiter le grain de la peau.

Les modèles de comparaison peuvent être réalisés chacun sur un support de forme générale rectangulaire et de dimensions voisines de 60 mm par 100 mm, par exemple.

Le nuancier peut se présenter sous la forme d'un ensemble de modèles de comparaison reliés en éventail et correspondant chacun à une couleur.

Le nuancier peut aussi comporter un ou plusieurs supports comportant chacun plusieurs modèles de comparaison.

Ainsi, le nuancier peut comporter au moins une bande comportant plusieurs modèles de comparaison, par exemple au moins cinq modèles de comparaison, juxtaposés.

Les modèles de comparaison sont avantageusement chacun réalisés avec un support comportant un trou, situé par exemple à environ un tiers de leur longueur, ce trou étant par exemple circulaire de diamètre voisin de 20 mm.

Chaque modèle de comparaison peut comporter un identifiant propre, tel qu'un code alphanumérique, de manière à permettre à l'utilisateur d'identifier aisément les différents modèles de comparaison.

Les modèles de comparaison peuvent présenter une brillance non homogène, notamment lorsqu'il s'agit d'imiter l'aspect de la peau, en particulier son caractère localement plus ou moins brillant.

Les modèles de comparaison peuvent ainsi comporter en surface une juxtaposition de zones élémentaires ayant des brillances différentes.

Les zones les plus brillantes peuvent devoir leur brillance à la présence d'un vernis brillant et les zones les moins brillantes peuvent devoir leur matité à la présence d'un vernis mat.

La largeur des zones les plus brillantes peut être voisine de 300 µm et celle des zones les moins brillantes voisine de 100 µm, lorsque les modèles de comparaison visent à imiter l'aspect de la peau.

D'une manière générale, chaque modèle de comparaison peut reproduire la couleur de la peau ou la couleur d'un autre élément kératinique que la peau et au moins une caractéristique d'apparence autre que la couleur, par exemple la brillance, le relief ou l'hétérogénéité de couleur.

Ainsi, le nuancier peut comporter au moins deux modèles de comparaison ayant des brillances différentes, des reliefs différents ou des répartitions de couleurs différentes au sein de chaque modèle.

Le caractère plus ou moins brillant peut être obtenu au moyen d'un vernis plus ou moins brillant.

Il peut encore être obtenu grâce à un relief déterminé en surface.

Les modèles de comparaison peuvent avoir une brillance homogène mais de préférence, comme mentionné plus haut, les modèles de comparaison ont une brillance hétérogène, notamment lorsqu'il s'agit d'imiter l'aspect de la peau.

Le nuancier peut comporter un seul modèle de comparaison rapporté ou imprimé sur un emballage.

L'invention a encore pour objet, parmi d'autres, un système comportant

- une pluralité de modèles de comparaison, chaque modèle de comparaison ayant un spectre de réflectance et étant configuré pour simuler sensiblement une couleur d'un élément kératinique ayant un spectre de réflectance,

  système dans lequel le spectre de réflectance de chaque modèle de comparaison est sensiblement similaire au spectre de réflectance d'un élément kératinique respectif, de telle sorte que le modèle de comparaison et l'élément kératinique apparaissent pour un observateur, comme ayant sensiblement la même couleur sous au moins deux illuminants différents.

Chaque modèle de comparaison peut être configuré pour présenter une brillance non uniforme. Chaque modèle de comparaison peut comporter des régions adjacentes ayant des brillances différentes. Chaque modèle de comparaison peut comporter au moins une région relativement brillante, cette région relativement brillante comportant un vernis brillant. Chaque modèle de comparaison peut comporter une région relativement mate, cette région relativement mate comportant un vernis mat. Chaque modèle de comparaison peut être configuré pour simuler au moins une caractéristique d'apparence autre que la couleur de l'élément kératinique. Chaque modèle de comparaison peut être configuré pour être affiché par l'intermédiaire d'une image électronique.

L'invention a encore pour objet un procédé de fabrication d'un modèle de comparaison configuré pour simuler sensiblement la couleur d'un élément kératinique, ce procédé comportant l'étape consistant à déposer un revêtement sur un support, le revêtement ayant un spectre de réflectance qui est sensiblement similaire à un spectre de réflectance de l'élément kératinique, de telle sorte que le modèle de comparaison et l'élément kératinique apparaissent pour un observateur comme ayant sensiblement la même couleur sous au moins deux illuminants différents.

L'élément kératinique peut être la peau, les cheveux, les ongles des pieds ou des mains.

L'invention a encore pour objet un procédé de fabrication d'un produit destiné à être appliqué sur un élément kératinique, ce procédé comportant les étapes suivantes :

- fournir un système tel que précité,
- sélectionner au moins un modèle de comparaison parmi la pluralité de modèles de comparaison,
- fabriquer un produit destiné à être appliqué sur un élément kératinique en fonction de la couleur dudit au moins un modèle de comparaison sélectionné.

Le système peut être fourni au moyen d'un support matériel ou sous une forme numérique, imprimé au moyen d'un dispositif d'impression recevant des informations numériques.

Le produit peut être notamment un fond de teint, un produit destiné à masquer les imperfections de la peau, un produit pour les lèvres, un produit de coloration capillaire, un produit de soins pour les cheveux, un vernis à ongles, un blush, une ombre à paupières, un produit de coloration de la peau ou un produit de soin de la peau.

L'invention a encore pour objet un procédé pour réaliser un nuancier regroupant une pluralité de modèles de comparaison reproduisant chacun la couleur d'un élément kératinique, notamment une couleur de peau, caractérisé par le fait que pour chaque modèle de comparaison, on dépose sur un support un revêtement ayant un spectre de réflectance suffisamment proche de celui de l'élément kératinique pour que la couleur de ce dernier et celle du modèle de comparaison apparaissent sensiblement identiques sous au moins deux illuminants différents.

De préférence, on fait correspondre le spectre de réflectance de chaque modèle de comparaison avec celui de l'élément kératinique correspondant sur tout le domaine spectral s'étendant entre 400 et 800 nm.

De préférence, l'un au moins du support et du revêtement subit un traitement destiné à lui permettre d'imiter la texture de la peau.

Un tel traitement peut comporter un embossage du support, voire du revêtement.

Le traitement peut également consister à imprimer des motifs sur un fond coloré, les motifs ayant une couleur différente de celle du fond.

Dans tous les cas, le choix des pigments et/ou colorants utilisés pour réaliser le revêtement sera effectué en fonction de l'incidence sur la couleur finale du traitement mis en oeuvre.

Le traitement peut encore consister à imprimer un vernis mat ou brillant, afin de conférer une brillance hétérogène au modèle de comparaison.

Le cas échéant, le modèle de comparaison peut recevoir une impression d'un vernis mat et une impression d'un vernis brillant.

L'invention a encore pour objet un procédé de fabrication d'un produit cosmétique ou de soins, notamment un fond de teint, caractérisé par le fait qu'il comprend les étapes suivantes :

- fournir un nuancier tel que défini plus haut,
- déterminer le modèle de comparaison correspondant à la couleur de la peau d'une personne ou à une couleur souhaitée,
- fabriquer un produit cosmétique, notamment un fond de teint, ayant la couleur du modèle de comparaison ainsi déterminé, en vue de son application sur la peau de cette personne.

Dans le procédé ci-dessus, le nuancier peut être fourni matériellement ou imprimé au moyen d'une imprimante adéquate à partir d'un fichier téléchargé.

L'invention a encore pour objet un procédé pour contrôler le degré de bronzage d'une personne, caractérisé par le fait qu'il comporte les étapes suivantes :

- déterminer parmi les modèles de comparaison du nuancier tel que défini plus haut celui dont la couleur est la plus proche de celle de la peau de la personne dont on cherche à évaluer le bronzage, ce modèle de comparaison servant de référence,
- exposer la peau de la personne à un rayonnement ultraviolet et/ou appliquer ou administrer un produit autobronzant ou photo-sensibilisant,
- comparer la couleur de la peau après cette exposition ou administration ou application avec celle du modèle de comparaison de référence et déterminer si un niveau de bronzage souhaité est atteint.

Ainsi, on peut éviter par exemple à une personne fréquentant un solarium de s'exposer inutilement au rayonnement ultraviolet une fois le degré de bronzage souhaité atteint ou proche d'être atteint.

Le procédé peut comporter l'étape consistant à sélectionner un régime de bronzage en fonction du modèle de comparaison sélectionné.

L'invention a encore pour objet un procédé pour suivre le traitement, notamment cosmétique, d'un élément kératinique avec un produit, le procédé comportant les étapes suivantes :

- fournir un système tel que défini plus haut,
- sélectionner un modèle de comparaison qui correspond sensiblement à une couleur de l'élément kératinique,
- appliquer un produit sur l'élément kératinique et déterminer si la couleur de l'élément kératinique sur lequel le produit a été appliqué a été modifiée après l'application du produit, en comparant l'élément kératinique et les modèles de comparaison.

Le procédé peut comporter une étape consistant à exposer l'élément kératinique à une radiation ultraviolette avant de déterminer si la couleur de l'élément kératinique sur lequel le produit a été appliqué a été modifiée.

Le produit peut être un produit cosmétique de protection solaire ou un produit auto-bronzant.

L'invention a encore pour objet un procédé pour sélectionner un produit destiné à être appliqué sur un élément kératinique, ce procédé comportant les étapes suivantes :

- fournir un système tel que défini plus haut,
- sélectionner un modèle de comparaison ayant une couleur qui correspond sensiblement à l'élément kératinique sur lequel le produit est destiné à être appliqué, et
- sélectionner un produit parmi une pluralité de produits différents destinés a être appliqués sur l'élément kératinique, en fonction du modèle de comparaison sélectionné.

Chacun des modèles de comparaison peut comporter un identifiant associé à la couleur du modèle, et la sélection du produit peut être fonction de l'identifiant du modèle de comparaison sélectionné.

Chacun des produits peut comporter un identifiant qui correspond à l'un des identifiants de l'un des modèles de comparaison et la sélection du produit peut consister à sélectionner le produit dont l'identifiant correspond à celui du modèle de comparaison.

L'invention a encore pour objet un procédé pour traiter un élément kératinique, le procédé comportant les étapes suivantes :

- fournir un système tel que défini plus haut,
- sélectionner un modèle de comparaison qui correspond à une couleur souhaitée pour l'élément kératinique,
- traiter l'élément kératinique en fonction du modèle de comparaison sélectionné.

Le traitement peut comporter l'étape consistant à appliquer un produit sur l'élément kératinique.

Le procédé peut comporter en outre la sélection d'un produit destiné à traiter l'élément kératinique à partir d'une pluralité de produits différents, en fonction du modèle de comparaison sélectionné.

Chaque modèle de comparaison peut comporter un identifiant associé à la couleur du modèle et la sélection du produit peut être fonction de l'identifiant du modèle de comparaison sélectionné.

L'invention a encore pour objet un procédé permettant l'analyse d'un élément kératinique, le procédé comportant les étapes suivantes :

- transmettre au moins une image ayant un spectre de réflectance et étant configurée pour simuler sensiblement une couleur de l'élément kératinique, ce dernier ayant un spectre de réflectance, le spectre de réflectance de l'image étant sensiblement similaire au spectre de réflectance de l'élément kératinique, de telle sorte que ladite au moins une image et l'élément kératinique apparaissent pour un observateur comme ayant sensiblement la même couleur sous au moins deux illuminants différents.

Le procédé peut comporter l'étape consistant à comparer l'élément kératinique analysé avec ladite au moins une image afin de déterminer si ladite au moins une image correspond sensiblement à la couleur de l'élément kératinique.

La transmission de l'image peut s'effectuer par l'intermédiaire d'un réseau, par exemple un réseau Internet ou Intranet.

Le procédé peut comporter en outre l'étape consistant à recevoir une information relative à une comparaison entre l'élément kératinique et ladite au moins une image.

L'invention a encore pour objet un procédé pour déterminer l'efficacité d'un produit cosmétique ou de soins, notamment une crème de protection solaire ou un produit autobronzant, caractérisé par le fait qu'il comporte les étapes suivantes :

- déterminer parmi les modèles de comparaison du nuancier tel que défini plus haut, celui dont la couleur est la plus proche de celle de la peau d'une personne donnée,
- appliquer sur la peau de cette personne le produit cosmétique ou de soin,
- éventuellement exposer la peau de cette personne à un rayonnement ultraviolet,
- rechercher une éventuelle variation de la couleur de la peau en procédant à une nouvelle comparaison avec les modèles de comparaison du nuancier.

Un tel procédé peut permettre de quantifier aisément la variation de couleur de la peau suite à un traitement donné, donc d'évaluer l'efficacité de celui-ci.

L'invention a encore pour objet l'utilisation du nuancier tel que défini plus haut pour déterminer l'état de santé d'un individu, en détectant à l'aide du nuancier une variation éventuelle anormale de la couleur de la peau de cet individu, caractéristique d'un état maladif par exemple.

L'invention a encore pour objet un procédé de sélection d'un produit cosmétique ou de soin, caractérisé par le fait qu'il comporte les étapes suivantes :

- faire figurer sur chaque emballage d'une même gamme de produits cosmétiques ou de soin ayant différentes couleurs des identifiants tels qu'un code alphanumérique spécifique à la couleur de chaque produit,
- faire figurer les mêmes identifiants sur les modèles de comparaison du nuancier tel que défini plus haut,

- déterminer parmi les modèles de comparaison du nuancier celui dont la couleur est la plus proche de celle de la peau d'un individu donné,
- sélectionner, en vue de l'application sur la peau de cet individu, le produit dont l'emballage comporte le même identifiant que celui figurant sur le modèle de comparaison précédemment déterminé.

L'invention a encore pour objet un procédé de fabrication d'un emballage comportant les étapes suivantes : fabriquer un emballage comportant au moins un modèle de comparaison reproduisant la couleur d'un élément kératinique, notamment de la peau, ce modèle de comparaison étant réalisé au moyen de pigments et/ou de colorants choisis de telle sorte que son spectre de réflectance présente un profil suffisamment proche de celui de l'élément kératinique dont il reproduit la couleur, pour que ce dernier et le modèle de comparaison correspondant apparaissent, pour un observateur, de la même couleur sous au moins deux illuminants différents.

L'invention a encore pour objet un procédé de traitement cosmétique, caractérisé par le fait qu'il comporte les étapes suivantes :

- fournir un nuancier tel que défini plus haut,
- déterminer la couleur d'un élément kératinique à l'aide dudit nuancier,
- effectuer un traitement cosmétique en fonction de la couleur précédemment déterminée.

On entend par traitement cosmétique tout traitement non thérapeutique au moyen d'un produit cosmétique tel que défini dans la Directive 76/768/CEE modifiée par la Directive 93/35/CEE du Conseil du 14 juin 1993, un maquillage étant un exemple de traitement cosmétique.

D'autres caractéristiques et avantages de la présente invention ressortiront à la lecture de la description détaillée qui va suivre, d'exemples de mise en oeuvre non limitatifs, et à l'examen du dessin annexé, sur lequel :

- la figure 1 représente schématiquement un nuancier conforme à un premier exemple de mise en oeuvre de l'invention,
- les figures 2 et 3 illustrent schématiquement deux variantes de mise en oeuvre de l'invention,
- la figure 4 illustre schématiquement l'utilisation d'un modèle de comparaison,
- la figure 5 représente le spectre de réflectance d'une peau et celui du modèle de comparaison correspondant,
- les figures 6 à 9 illustrent schématiquement différentes structures de revêtement ou de support permettant de restituer une brillance hétérogène,

- la figure 10 représente de manière très schématique un système permettant l'impression à distance d'un modèle de comparaison,
- la figure 11 représente un support en forme de bande comprenant cinq modèles de comparaison, et
- la figure 12 est une vue schématique illustrant un exemple d'agencement relatif des zones de brillances différentes.

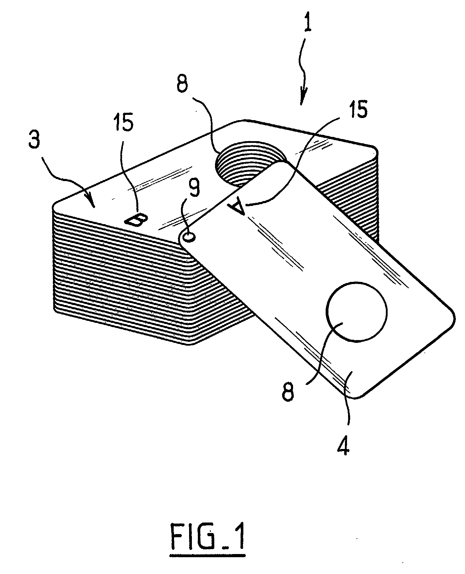

On a représenté sur la figure 1 un nuancier 1 conforme à l'invention, ce nuancier comportant plusieurs modèles de comparaison 4 reproduisant chacun une couleur de peau.

Chaque modèle de comparaison 4 comporte, dans l'exemple décrit, un support recouvert d'un revêtement coloré.

Chaque modèle de comparaison présente une forme sensiblement rectangulaire, de dimensions voisines de 60 mm par 100 mm dans l'exemple décrit.

Chaque modèle de comparaison 4 est traversé par un trou 8, ici circulaire de diamètre égal à 20 mm environ et situé, dans l'exemple décrit, à environ un tiers de sa longueur.

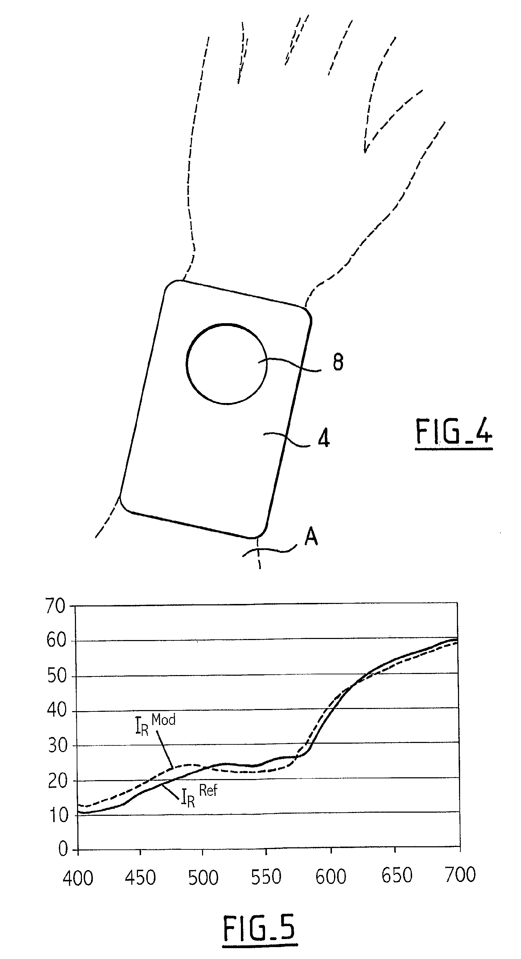

Un tel trou 8 permet, lorsqu'un modèle de comparaison 4 est posé sur une partie du corps ou du visage, par exemple l'avant-bras A comme illustré sur la figure 4, d'observer simultanément l'apparence de la peau et celle du modèle de comparaison 4, ce qui facilite la comparaison.

La forme rectangulaire du modèle de comparaison 4 permet éventuellement, par rapport à une forme carrée ou circulaire, d'orienter le modèle de comparaison 4 dans une direction particulière au moment de l'utilisation, ce qui s'avère préférable si le modèle de comparaison imite la texture de la peau, laquelle est anisotropique.

Chaque modèle de comparaison 4 comporte un identifiant 15 constitué par exemple par un ou plusieurs caractères alphanumériques.

Dans l'exemple décrit, le nuancier 1 comporte cinquante modèles de comparaison 4 correspondant à cinquante couleurs de peau différentes.

Tous les modèles de comparaison 4 présentent ici la même brillance et la même texture.

Les cinquante couleurs résultent, dans l'exemple décrit, de la combinaison de cinq teintes et de dix niveaux de clarté.

Les angles de teinte (h), mesurés dans l'espace CIEL*C*h 1976, sont compris entre 40 et 70, préférentiellement entre 46 et 64.

Les niveaux de clarté L* dans l'espace CIEL*C*h 1976 sont compris dans l'exemple décrit entre 25 et 80, préférentiellement entre 30 et 70.

Les niveaux de chroma sont préférentiellement compris entre 12 et 30 et de préférence autour de 22.

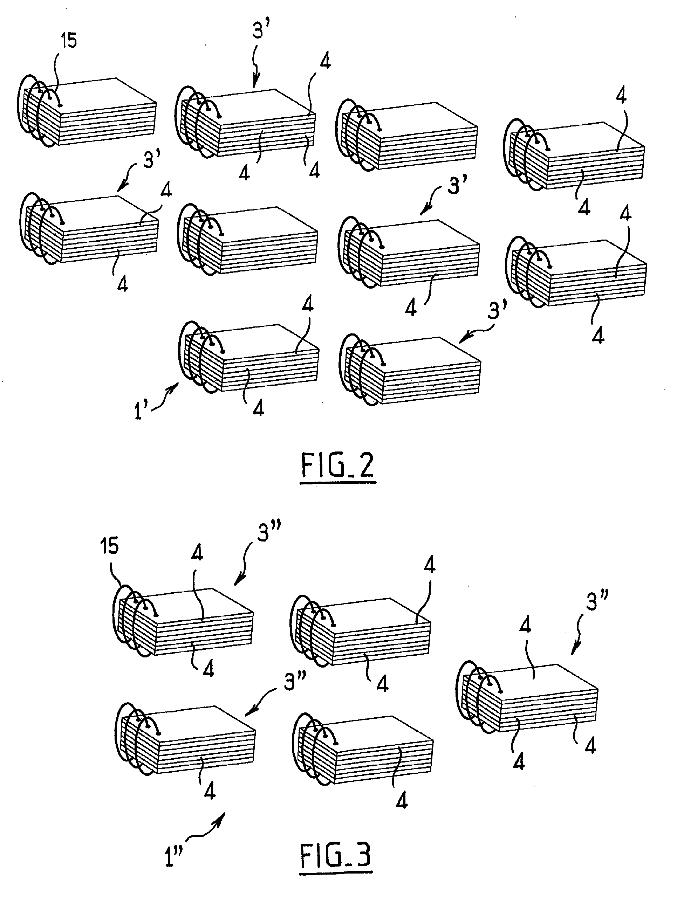

Les modèles de comparaison 4 peuvent être reliés en éventail en étant articulés autour d'un axe 9 comme cela est représenté sur la figure 1 ou être reliés au moyen de spires 15, comme cela est représenté sur les figures 2 et 3.

Par ailleurs, les modèles de comparaison 4 du nuancier peuvent être regroupés de plusieurs façons.

Tous les modèles de comparaison 4 peuvent être reliés ensemble au sein d'une liasse 3 unique, comme représenté sur la figure 1.

On peut encore constituer plusieurs groupes indépendants, afin de faciliter la manipulation des modèles de comparaison.

Le nuancier 1' représenté à la figure 2 comporte dix liasses 3' correspondant chacune à une teinte déterminée, différente de celle des autres liasses, chaque liasse 3 comportant cinq modèles de comparaison 4 de niveaux de clarté différents mais de même teinte.

Le nuancier 1" représenté à la figure 3 comprend cinq liasses 3" correspondant chacune à une teinte déterminée, différente de celle des autres liasses, chaque liasse 3" comportant dix modèles de comparaison 4 de clartés différentes mais de même teinte.

D'autres regroupements peuvent encore être effectués sans que l'on sorte du cadre de la présente invention.

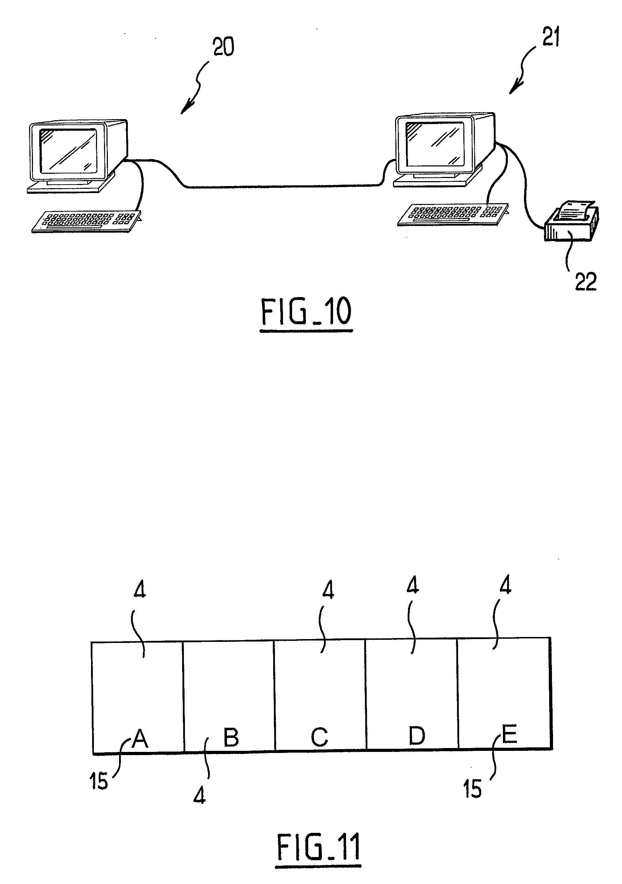

A titre d'exemple, on a représenté sur la figure 11 cinq modèles de comparaison juxtaposés sur un même support en forme de bande.

Ces cinq modèles de comparaison peuvent avoir par exemple même teinte et des luminosités différentes ou même luminosité mais des teintes différentes.

L'écart de couleur visuel mesuré dans l'espace colorimétrique CIEL\*C\*h 1976 entre deux modèles de comparaison 4 de couleurs voisines est de préférence constant, par exemple égal à 4, cette valeur permettant à une personne non entraînée de percevoir aisément une variation de couleur entre deux modèles de comparaison 4.

Les modèles de comparaison 4 permettent de reproduire fidèlement la couleur de la peau quelque soit l'illuminant, notamment que ce soit la lumière du jour ou un éclairage artificiel de type à incandescence ou fluorescence.

D'une manière générale, on cherche à faire correspondre au mieux le spectre de réflectance du modèle de comparaison avec celui de la peau correspondante, dans la gamme de longueurs d'onde 400-800 nm.

Les modèles de comparaison 4 sont ainsi fabriqués à partir de la connaissance des spectres de réflectance de toutes les variétés de peaux caucasiennes, noires ou asiatiques notamment.

A titre d'exemple, on a représenté en trait plein sur la figure 5 l'intensité relative refléchie $I_R^{REF}$ (en %) en fonction de la longueur d'onde (en nm) pour une peau donnée et, en trait interrompu, l'intensité relative réfléchie $I_R^{MOD}$ (en %) en fonction de la longueur d'onde (en nm) pour le modèle de comparaison correspondant.

De préférence, $\Delta$ $1/N(\lambda)$ $\sum_{\lambda}$$| I_R^{MOD} - I_R^{REF} |$

$/I_R^{REF} \leq 0,1$ préférentiellement $\leq 0,05$ et de préférence encore $\leq 0,01$.

Il est possible de privilégier des sous-intervalles spectraux dans lesquels les spectres de réflectance du modèle de comparaison 4 et de la peau correspondante sont beaucoup plus proches, c'est-à-dire $\Delta$ plus petit.

On peut ainsi privilégier, par exemple, l'intervalle spectral [600 nm ; 750 nm] correspondant à la couleur rouge et à ses nuances.

On peut avoir, par exemple, $\Delta \leq 0,01$ dans cet intervalle spectral.

Pour obtenir le spectre de réflectance recherché pour chaque modèle de comparaison 4, on peut se servir de logiciels connus permettant de déterminer une composition pigmentaire correspondant à un spectre de réflectance donné.

On peut par exemple utiliser le logiciel DATAMATCH de la société DATACOLOR INTERNATIONAL.

La couleur des modèles de comparaison 4 peut être homogène.

En variante, on peut réaliser des modèles de comparaison 4 présentant chacun une couleur hétérogène, c'est-à-dire présentant des variations locales de teinte et/ou de clarté.

La réflectance spectrale du modèle de comparaison 4 correspond alors à une valeur moyenne, pour une surface de 1 cm de diamètre, par exemple.

Des motifs peuvent être réalisés sur les modèles de comparaison 4 afin d'imiter la texture de la peau.

Chaque modèle de comparaison 4 peut ainsi comporter des zones de teintes et/ou de clartés différentes, par exemple obtenues par impression de motifs avec une couleur différente de celle du fond, par exemple une couleur bistre.

Il est également possible d'imiter l'aspect de la peau en utilisant un support présentant un relief, par exemple un support embossé de manière à imiter le grain de la peau.

L'embossage peut être réalisé par exemple par calandrage, avant ou après le dépôt du revêtement coloré.

Pour mieux imiter l'apparence de la peau, et notamment son caractère localement plus ou moins brillant, il est souhaitable de conférer aux modèles de comparaison 4 une brillance non homogène.

Il est possible d'obtenir une brillance non homogène de plusieurs manières.

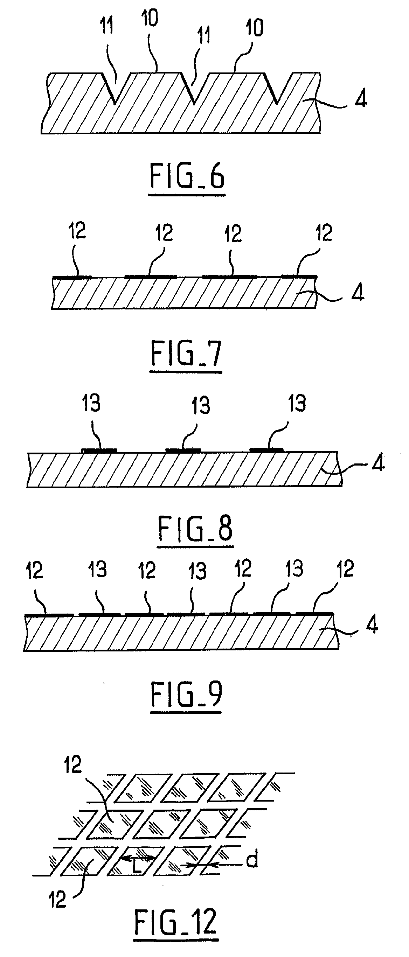

On peut notamment réaliser sur le support des plateaux 10 et des creux 11, comme cela est représenté sur la figure 6.

Les zones du revêtement coloré qui épousent la forme des creux 11 apparaissent alors moins brillantes que celles recouvrant les plateaux 10.

En variante, ou additionnellement, on peut appliquer sur les plateaux 10 un vernis brillant, les creux 11 en étant dépourvus.

On peut encore obtenir une brillance non homogène en appliquant sur un support plan, comme cela est illustré sur les figures 7 et 12, un vernis brillant 12 sur une partie seulement de la surface du modèle de comparaison, par exemple sous la forme de carrés L = 300 µm de largeur environ, espacés entre eux d'une distance d = 100 µm environ.

En variante, comme cela est illustré sur la figure 8, on peut appliquer un vernis mat 13, par exemple sous la forme de zones de 100 µm de largeur, espacées entre elles d'une distance de 300 µm environ.

On peut encore juxtaposer ou superposer un vernis brillant et un vernis mat, les zones 12 de vernis brillant ayant une largeur voisine de 300 µm et les zones 13 de vernis mat ayant une largeur voisine de 100 µm, comme cela est représenté sur la figure 9.

Les nuanciers représentés aux figures 1, 2 et 3 peuvent comporter des modèles de comparaison ayant une caractéristique d'apparence autre que la couleur qui varie d'un modèle à l'autre.

En particulier, la brillance peut varier d'un modèle à l'autre afin de traduire le fait qu'il existe des peaux plus grasses que d'autres.

Dans ce cas, un utilisateur du nuancier déterminera non seulement la couleur de la peau mais également la brillance correspondant à celle-ci.

D'autres caractéristiques d'apparence telles que le relief ou la distribution de la brillance ou de la couleur au sein de chaque modèle peuvent encore varier.

Le nuancier peut trouver de nombreuses utilisations, dans le domaine de la cosmétique notamment.

Tout d'abord, le nuancier est utile pour effectuer des études statistiques dans la population, afin d'en extraire des caractéristiques typologiques par exemple.

Par ailleurs, le nuancier permet à une personne de connaître précisément la couleur de sa peau, ce qui peut faciliter ensuite l'achat d'un produit cosmétique, notamment un fond de teint, cette personne n'ayant qu'à choisir le fond de teint affecté du même identifiant que celui présent sur le modèle de comparaison pour être sûre d'avoir la bonne couleur.

Le nuancier peut également être utile pour une personne qui cherche à s'appliquer un produit qui ne correspond pas exactement à la couleur de sa peau, mais présente une luminosité et/ou une teinte différente, correspondant à un modèle de comparaison donné du nuancier.

Dans ce cas, de par le positionnement relatif de ce modèle de comparaison par rapport au modèle de comparaison correspondant à la couleur de la peau de l'utilisateur, ce dernier pourra déterminer comment une couleur qui l'intéresse se situe en termes de clarté et de teinte par rapport à la couleur de sa peau.

Le nuancier selon l'invention est également utile pour déterminer une variation de la couleur de la peau, suite au traitement par un produit ou tout simplement suite à l'exposition au soleil ou à une source artificielle de rayonnement ultraviolet.

Le nuancier peut permettre de déterminer si le degré de bronzage souhaité est atteint pour une personne donnée.

Dans l'affirmative, cette personne est informée qu'une exposition ultérieure ne sera pas nécessaire, ce qui permet d'éviter une exposition excessive ou d'adopter une protection solaire.

Le nuancier peut également être utilisé pour déterminer l'effet sur la couleur de la peau d'un produit cosmétique ou de soin, par exemple un produit auto-bronzant.

Dans ce cas, l'utilisateur peut déterminer l'efficacité du traitement par comparaison avec un modèle de comparaison servant de référence et correspondant à la couleur de la peau avant traitement.

Le nuancier peut être proposé sous une forme matérielle telle que celle représentée sur les figures 1 à 3.

Toutefois, on ne sort pas du cadre de la présente invention lorsque le nuancier est fourni à l'utilisateur sous une forme non matérielle, par exemple sous la forme d'un fichier de données informatiques contenant toutes les informations pour permettre l'impression ou l'affichage des modèles de comparaison sur un support adapté.

A titre d'exemple, on a illustré sur la figure 10 le transfert depuis un premier ordinateur 20 vers un second ordinateur 21 d'un fichier de données informatiques contenant les informations nécessaires pour l'impression, au moyen d'une imprimante adaptée 22, des différents modèles de comparaison.

La transmission des données entre les ordinateurs 20 et 21 peut s'effectuer par exemple par un réseau informatique tel qu'Internet.

Bien sûr, l'invention n'est pas limitée aux exemples de mise en oeuvre qui viennent d'être décrits.

On peut notamment réaliser des nuanciers reproduisant fidèlement la couleur d'une lèvre, d'un ongle ou de cheveux d'un type déterminé.

## Revendications

1. Nuancier (1) comportant au moins un modèle de comparaison (4) reproduisant la couleur d'un élément kératinique, notamment de la peau, **caractérisé par le fait que** le ou chaque modèle de comparaison est réalisé au moyen de pigments et/ou de colorants choisis de telle sorte que son spectre de réflectance présente un profil suffisamment proche de celui de l'élément kératinique dont il reproduit la couleur pour que ce dernier et le modèle de comparaison correspondant apparaissent, pour un observateur, de la même couleur sous au moins deux illuminants différents.

2. Nuancier selon la revendication précédente, **caractérisé par le fait que** chaque modèle de comparaison (4) reproduit fidèlement la couleur de l'élément kératinique au moins sous deux des illuminants D65, D50 et A.

3. Nuancier selon l'une des deux revendications précédentes, **caractérisé par le fait que** le spectre de réflectance de chaque modèle de comparaison (4) reste proche de celui de l'élément kératinique dans un domaine spectral s'étendant de 400 à 800 nm.

4. Nuancier selon l'une quelconque des revendications précédentes, **caractérisé par le fait que**

$$1/N(\lambda) \sum_{\lambda} \left| I_R^{MOD}(\lambda) - I_R^{REF}(\lambda) \right| / I_R^{REF}(\lambda),$$

où $I_R^{MOD}(\lambda)$ est l'intensité lumineuse réfléchie à la longueur d'onde $\lambda$ pour le modèle de comparaison et $I_R^{REF}(\lambda)$ est l'intensité lumineuse réfléchie à la longueur d'onde $\lambda$ pour l'élément kératinique est inférieur ou égal à 0,1, de préférence $\leq$ 0,05 et de préférence encore $\leq$ 0,01.

5. Nuancier selon l'une quelconque des revendications précédentes, chaque modèle de comparaison reproduisant une couleur de peau, **caractérisé par le fait que** les modèles de comparaison (4) présentent des teintes différentes, les angles de teinte étant de préférence compris entre 40° et 70° et de préférence encore entre 46° et 64° dans l'espace colorimétrique CIEL*C*h 1976.

6. Nuancier selon la revendication précédente, **caractérisé par le fait qu'**il comporte au moins cinq catégories de modèles de comparaison présentant chacune une teinte déterminée, différente de celle des autres catégories.

7. Nuancier selon l'une quelconque des revendications précédentes, chaque modèle de comparaison reproduisant une couleur de peau, **caractérisé par le fait que** les modèles de comparaison (4) présentent des niveaux de clarté différents, les niveaux de clarté (L* dans l'espace colorimétrique CIEL*C*h 1976) étant de préférence compris entre 34 et 75.

8. Nuancier selon l'une quelconque des revendications précédentes, chaque modèle de comparaison reproduisant une couleur de peau, **caractérisé par le fait que** les modèles de comparaison présentent des niveaux de chroma différents, les niveaux de chroma étant de préférence compris entre 12 et 30, et de préférence autour de 22.

9. Nuancier selon l'une des deux revendications immédiatement précédentes, **caractérisé par le fait qu'**il comporte au moins dix catégories de modèles de comparaison présentant chacune un niveau de clarté déterminé, différent de celui des autres catégories.

10. Nuancier selon l'une quelconque des revendications précédentes, **caractérisé par le fait que** l'écart de couleur global $\Delta E*C*h.94$ mesuré dans l'espace colorimétrique CIEL*C*h 1976, entre deux modèles de comparaison correspondant à des couleurs de peau voisines, est constant, cet écart étant de préférence compris entre 1 et 40, de préférence 1 et 20, et de préférence encore voisin de 4.

11. Nuancier selon l'une quelconque des revendications précédentes, **caractérisé par le fait qu'**au sein d'un même modèle de comparaison, la couleur est uniforme et constante sur l'ensemble de la surface du modèle de comparaison.

12. Nuancier selon l'une quelconque des revendications 1 à 10, **caractérisé par le fait qu'**au sein d'un même modèle de comparaison, la couleur est non uniforme.

13. Nuancier selon la revendication précédente, **caractérisé par le fait qu'**un modèle de comparaison reçoit deux revêtements colorés de couleurs différentes.

14. Nuancier selon l'une quelconque des revendications précédentes, **caractérisé par le fait que** les modèles de comparaison (4) sont chacun réalisés sur un support de forme générale rectangulaire et de dimensions voisines de 60 mm par 100 mm de préférence.

15. Nuancier selon l'une quelconque des revendications précédentes, **caractérisé par le fait que** les modèles de comparaison (4) sont chacun réalisés sur un support comportant un trou (8), situé de préférence à environ un tiers de leur longueur, ce trou étant de préférence circulaire de diamètre voisin de 20 mm.

16. Nuancier selon l'une quelconque des revendications précédentes, **caractérisé par le fait que** chaque modèle de comparaison comporte un identifiant propre tel qu'un code alphanumérique.

17. Nuancier selon l'une quelconque des revendications précédentes, **caractérisé par le fait que** les modèles de comparaison présentent une brillance non homogène.

18. Nuancier selon la revendication précédente, **caractérisé par le fait que** les modèles de comparaison (4) comportent en surface une juxtaposition de zones élémentaires (12, 13) ayant des brillances différentes.

**19.** Nuancier selon la revendication précédente, **caractérisé par le fait que** les zones les plus brillantes doivent leur brillance à la présence d'un vernis brillant (12).

**20.** Nuancier selon la revendication précédente, **caractérisé par le fait que** la largeur (L) des zones les plus brillantes est voisine de 300 μm.

**21.** Nuancier selon l'une des trois revendications immédiatement précédentes, **caractérisé par le fait que** les zones les moins brillantes doivent leur matité à la présence d'un vernis mat (13).

**22.** Nuancier selon la revendication précédente, **caractérisé par le fait que** la largeur (d) des zones les moins brillantes est voisine de 100 μm.

**23.** Nuancier selon l'une quelconque des revendications précédentes, **caractérisé par le fait que** chaque modèle de comparaison reproduit une couleur et une caractéristique d'apparence autre que la couleur.

**24.** Nuancier selon la revendication précédente, **caractérisé par le fait que** la caractéristique d'apparence autre que la couleur est la brillance.

**25.** Nuancier selon la revendication 23, **caractérisé par le fait que** la caractéristique d'apparence autre que la couleur est le relief.

**26.** Nuancier selon la revendication 23, **caractérisé par le fait que** la caractéristique d'apparence autre que la couleur est l'hétérogénéité de couleur.

**27.** Nuancier selon la revendication 24, **caractérisé par le fait qu'**il comporte au moins deux modèles de comparaison (4) ayant des brillances différentes.

**28.** Nuancier selon l'une quelconque des revendications précédentes, **caractérisé par le fait que** les modèles de comparaison (4) comportent un relief déterminé leur conférant une brillance inhomogène.

**29.** Nuancier selon l'une quelconque des revendications précédentes, **caractérisé par le fait qu'**il comporte plusieurs modèles de comparaison reliés en éventail.

**30.** Nuancier selon l'une quelconque des revendications précédentes, **caractérisé par le fait qu'**il comporte plusieurs modèles de comparaison sur un même support, de préférence en forme de bande.

**31.** Nuancier selon l'une quelconque des revendications précédentes, **caractérisé par le fait qu'**il comporte un seul modèle de comparaison rapporté ou imprimé sur un emballage.

**32.** Procédé pour réaliser un nuancier regroupant une pluralité de modèles de comparaison reproduisant chacun la couleur d'un élément kératinique, notamment une couleur de peau, **caractérisé par le fait que**, pour chaque modèle de comparaison, on dépose sur un support un revêtement ayant un spectre de réflectance suffisamment proche de celui de l'élément kératinique pour que la couleur de l'élément kératinique et celle du modèle de comparaison apparaissent sensiblement identiques sous au moins deux illuminants différents.

**33.** Procédé selon la revendication précédente, **caractérisé par le fait que** l'on fait correspondre le spectre de réflectance de chaque modèle de comparaison avec celui de l'élément kératinique correspondant sur tout le domaine spectral s'étendant entre 400 et 800 nm.

**34.** Procédé selon l'une des revendications 32 et 33, **caractérisé par le fait que** l'un au moins du support et du revêtement subit un traitement destiné à lui permettre d'imiter la texture de la peau.

**35.** Procédé selon la revendication précédente, **caractérisé par le fait que** le traitement comporte un embossage du support, voire du revêtement.

**36.** Procédé selon la revendication 34, **caractérisé par le fait que** le traitement consiste à imprimer des motifs sur un fond coloré, les motifs ayant une couleur différente de celle du fond.

**37.** Procédé selon la revendication 34, **caractérisé par le fait que** le traitement consiste à imprimer un vernis mat ou brillant, afin de conférer une brillance hétérogène.

**38.** Procédé selon la revendication 34, **caractérisé par le fait que** le traitement consiste à imprimer un vernis mat et un vernis brillant.

**39.** Procédé de fabrication d'un produit cosmétique ou de soins, notamment un fond de teint, **caractérisé par le fait qu'**il comprend les étapes suivantes :

- fournir un nuancier selon l'une quelconque des revendications 1 à 31,
- déterminer le modèle de comparaison correspondant à la couleur de la peau d'une personne ou à une couleur souhaitée,
- fabriquer un produit cosmétique, notamment un fond de teint, ayant la couleur du modèle de comparaison ainsi déterminé, en vue de son application sur la peau de cette personne.

**40.** Procédé selon la revendication précédente, **caractérisé par le fait que** le nuancier est fourni matériellement.

**41.** Procédé selon la revendication 39, **caractérisé par le fait que** le nuancier est imprimé au moyen d'une imprimante adéquate à partir d'un fichier téléchargé.

**42.** Procédé pour contrôler le degré de bronzage d'une personne, **caractérisé par le fait qu'**il comporte les étapes suivantes :

- déterminer parmi les modèles de comparaison du nuancier selon l'une quelconque des revendications 1 à 31 celui dont la couleur est la plus proche de celle de la peau de la personne dont on cherche à évaluer le bronzage, ce modèle de comparaison servant de référence,
- exposer la peau de la personne à un rayonnement ultraviolet et/ou administrer et/ou appliquer un produit autobronzant et/ou photo-sensibilisant,
- comparer la couleur de la peau après cette exposition ou application ou administration avec celle du modèle de comparaison de référence et déterminer si un niveau de bronzage souhaité est atteint.

**43.** Procédé pour déterminer l'efficacité d'un produit cosmétique ou de soins, notamment une crème de protection solaire ou un produit autobronzant, **caractérisé par le fait qu'**il comporte les étapes suivantes :

- déterminer parmi les modèles de comparaison du nuancier tel que défini dans l'une quelconque des revendications 1 à 31 celui dont la couleur est la plus proche de celle de la peau d'une personne donnée,
- appliquer sur la peau de cette personne le produit cosmétique ou de soin,
- éventuellement exposer la peau de cette personne à un rayonnement ultraviolet,
- rechercher une éventuelle variation de la couleur de la peau en procédant à une nouvelle comparaison avec les modèles de comparaison du nuancier.

**44.** Procédé pour sélectionner un produit destiné à être appliqué sur un élément kératinique, ce procédé comportant les étapes suivantes :

- fournir un nuancier tel que défini dans l'une quelconque des revendications 1 à 31,

- sélectionner un modèle de comparaison ayant une couleur qui correspond sensiblement à l'élément kératinique sur lequel le produit est destiné à être appliqué, et
- sélectionner un produit parmi une pluralité de produits différents destinés à être appliqués sur l'élément kératinique, en fonction du modèle de comparaison sélectionné.

**45.** Procédé selon la revendication 44, **caractérisé par le fait que** chacun des modèles de comparaison comporte un identifiant associé à la couleur du modèle, et **par le fait que** la sélection du produit est fonction de l'identifiant du modèle de comparaison sélectionné.

**46.** Procédé selon la revendication 44, **caractérisé par le fait que** chacun des produits comporte un identifiant qui correspond à l'un des identifiants de l'un des modèles de comparaison et **par le fait que** la sélection du produit consiste à sélectionner le produit dont l'identifiant correspond à celui du modèle de comparaison.

**47.** Procédé de fabrication d'un emballage, comportant les étapes suivantes :

fabriquer un emballage comportant au moins un modèle de comparaison reproduisant la couleur d'un élément kératinique, notamment de la peau, ce modèle de comparaison étant réalisé au moyen de pigments et/ou de colorants choisis de telle sorte que son spectre de réflectance présente un profil suffisamment proche de celui de l'élément kératinique dont il reproduit la couleur, pour que ce dernier et le modèle de comparaison correspondant apparaissent, pour un observateur, de la même couleur sous au moins deux illuminants différents.

**48.** Procédé de traitement cosmétique, **caractérisé par le fait qu'**il comporte les étapes suivantes :

- fournir un nuancier tel que défini dans l'une quelconque des revendications 1 à 31,
- déterminer la couleur d'un élément kératinique à l'aide dudit nuancier,
- effectuer un traitement cosmétique en fonction de la couleur précédemment déterminée.

**49.** Procédé pour suivre le traitement, notamment cosmétique, d'un élément kératinique avec un produit, le procédé comportant les étapes suivantes :

- fournir un nuancier tel que défini dans l'une quelconque des revendications 1 à 31,
- sélectionner un modèle de comparaison qui correspond sensiblement à une couleur de l'élément kératinique,

- appliquer un produit sur l'élément kératinique et déterminer si la couleur de l'élément kératinique sur lequel le produit a été appliqué a été modifiée après l'application du produit, en comparant l'élément kératinique et les modèles de comparaison.

50. Procédé selon la revendication 49, **caractérisé par le fait que** le produit est un produit cosmétique de protection solaire ou un produit auto-bronzant.

51. Procédé pour traiter un élément kératinique, comportant les étapes suivantes :

- fournir un nuancier tel que défini dans l'une quelconque des revendications 1 à 31,
- sélectionner un modèle de comparaison qui correspond à une couleur souhaitée pour l'élément kératinique,
- traiter l'élément kératinique en fonction du modèle de comparaison sélectionné.

52. Procédé selon la revendication précédente, **caractérisé par le fait que** le traitement comporte l'étape consistant à appliquer un produit sur l'élément kératinique.

53. Procédé selon la revendication 51 ou 52, **caractérisé par le fait qu'**il comporte en outre la sélection d'un produit destiné à traiter l'élément kératinique à partir d'une pluralité de produits différents, en fonction du modèle de comparaison sélectionné.

54. Procédé selon l'une quelconque des revendications 51 à 53, **caractérisé par le fait que** chaque modèle de comparaison comporte un identifiant associé à la couleur du modèle et **par le fait que** la sélection du produit est fonction de l'identifiant du modèle de comparaison sélectionné.

55. Procédé permettant l'analyse d'un élément kératinique, comportant l'étape suivante :

- transmettre au moins une image ayant un spectre de réflectance et étant configurée pour simuler sensiblement une couleur de l'élément kératinique, ce dernier ayant un spectre de réflectance, le spectre de réflectance de l'image étant sensiblement similaire au spectre de réflectance de l'élément kératinique, de telle sorte que ladite au moins une image et l'élément kératinique apparaissent pour un observateur comme ayant sensiblement la même couleur sous au moins deux illuminants différents.

56. Procédé selon la revendication précédente, **caractérisé par le fait qu'**il comporte l'étape consistant à comparer l'élément kératinique analysé avec ladite au moins une image afin de déterminer si ladite au moins une image correspond sensiblement à la couleur de l'élément kératinique.

57. Procédé selon l'une des revendications 55 et 56, **caractérisé par le fait que** la transmission de l'image s'effectue par l'intermédiaire d'un réseau, notamment un réseau Internet ou Intranet.

58. Procédé selon l'une quelconque des revendications 55 à 57, **caractérisé par le fait qu'**il comporte en outre l'étape consistant à recevoir une information relative à une comparaison entre l'élément kératinique et ladite au moins une image.

FIG.1

FIG. 2

FIG. 3

FIG_4

FIG_5

FIG_6

FIG_7

FIG_8

FIG_9

FIG_12

FIG_10

FIG_11

**Office européen
des brevets**

# RAPPORT DE RECHERCHE EUROPEENNE

Numéro de la demande

EP 01 40 3167

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | US 5 311 293 A (BILLMEYER FRED W  ET AL) 10 mai 1994 (1994-05-10) | 44-46,55 | A45D44/00 |
| A | | 1-5 | |
| | * colonne 1, ligne 18 - ligne 24 * <br> * colonne 5, ligne 15 - ligne 22 * <br> * colonne 5, ligne 52 - colonne 6, ligne 17 * | | |
| A | FR 2 540 991 A (SIGNOREL YVES) 17 août 1984 (1984-08-17) | 1,32,39, 42-49, 51,55 | |
| | * page 1, ligne 1 - ligne 5 * <br> * page 1, ligne 12 - ligne 14 * <br> * page 1, ligne 31 - page 2, ligne 40 * <br> * revendications 1,12 * <br> * figures 2,3 * | | |
| A | US 1 741 080 A (STENZ BERNARD F) 24 décembre 1929 (1929-12-24) <br> * page 1, ligne 1 - ligne 12 * <br> * page 1, ligne 60 - ligne 73 * <br> * figures 1-3 * | 1,44,45 | |
| A | PATENT ABSTRACTS OF JAPAN vol. 017, no. 457 (P-1597), 20 août 1993 (1993-08-20) & JP 05 107115 A (KAZUMI TAWARA), 27 avril 1993 (1993-04-27) * abrégé * | 1 | **DOMAINES TECHNIQUES RECHERCHES (Int.Cl.7)** <br> A45D <br> G01J <br> G01N <br> G09F <br> A61B |
| A | PATENT ABSTRACTS OF JAPAN vol. 1997, no. 09, 30 septembre 1997 (1997-09-30) & JP 09 133584 A (KAO CORP), 20 mai 1997 (1997-05-20) * abrégé * | 1 | |

-/--

Le présent rapport a été établi pour toutes les revendications

| Lieu de la recherche | Date d'achèvement de la recherche | Examinateur |
|---|---|---|
| LA HAYE | 19 mars 2002 | Amaro, H |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen
des brevets

**RAPPORT DE RECHERCHE EUROPEENNE**

Numéro de la demande

EP 01 40 3167

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | US 5 852 675 A (HAYASHI MASAHO ET AL) 22 décembre 1998 (1998-12-22) <br> * colonne 1, ligne 6 - ligne 30 * <br> * colonne 1, ligne 58 - colonne 2, ligne 46 * <br> * colonne 6, ligne 44 - ligne 59 * <br> * figures 1,2 * | 1,55 | |

DOMAINES TECHNIQUES RECHERCHES (Int.Cl.7)

Le présent rapport a été établi pour toutes les revendications

| Lieu de la recherche | Date d'achèvement de la recherche | Examinateur |
|---|---|---|
| LA HAYE | 19 mars 2002 | Amaro, H |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................
& : membre de la même famille, document correspondant

EPO FORM 1503 03 82 (P04C02)

**ANNEXE AU RAPPORT DE RECHERCHE EUROPEENNE
RELATIF A LA DEMANDE DE BREVET EUROPEEN NO.**     EP 01 40 3167

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

19-03-2002

| Document brevet cité au rapport de recherche | | Date de publication | Membre(s) de la famille de brevet(s) | | Date de publication |
|---|---|---|---|---|---|
| US 5311293 | A | 10-05-1994 | EP | 0682236 A1 | 15-11-1995 |
| | | | WO | 9530885 A1 | 16-11-1995 |
| | | | AU | 7092394 A | 29-11-1995 |
| | | | BR | 9408579 A | 06-05-1997 |
| | | | AT | 209777 T | 15-12-2001 |
| | | | DE | 69429248 D1 | 10-01-2002 |
| | | | EP | 1118845 A2 | 25-07-2001 |
| | | | EP | 1118846 A2 | 25-07-2001 |
| | | | US | 5313267 A | 17-05-1994 |
| | | | US | 5671735 A | 30-09-1997 |
| | | | US | 6067504 A | 23-05-2000 |
| | | | AU | 705197 B2 | 20-05-1999 |
| | | | JP | 10502729 T | 10-03-1998 |
| | | | KR | 220769 B1 | 15-09-1999 |
| FR 2540991 | A | 17-08-1984 | FR | 2540991 A1 | 17-08-1984 |
| US 1741080 | A | 24-12-1929 | AUCUN | | |
| JP 05107115 | A | 27-04-1993 | JP | 2011229 C | 02-02-1996 |
| | | | JP | 7035984 B | 19-04-1995 |
| JP 09133584 | A | 20-05-1997 | AUCUN | | |
| US 5852675 | A | 22-12-1998 | JP | 9005164 A | 10-01-1997 |

EPO FORM P0460

Pour tout renseignement concernant cette annexe : voir Journal Officiel de l'Office européen des brevets, No.12/82